# EUROPEAN PATENT APPLICATION

(11) **EP 3 628 315 A1**
(43) Date of publication of application: **01.04.2020**
(21) Application number: 18306278.5
(22) Date of filing: 28.09.2018
(51) Int. Cl.: A61K 31/166, A61K 31/27, A61K 31/404, A61K 31/439, A61K 31/445, A61K 31/4468, A61K 31/4525, A61K 31/473, A61K 31/5375, A61K 31/55, A61P 25/28

(54) **COMBINATION OF ACETYLCHOLINESTERASE INHIBITOR AND 5-HT4 RECEPTOR AGONIST AS NEUROPROTECTIVE AGENT IN THE TREATMENT OF NEURODEGENERATIVE DISEASES**

(71) Applicant: Université de Caen Normandie, 14000 Caen (FR); INSTITUT NATIONAL DE LA SANTE ET DE LA RECHERCHE MEDICALE (INSERM), 75013 Paris (FR); CENTRE NATIONAL DE LA RECHERCHE SCIENTIFIQUE, 75016 Paris (FR); Université de Montpellier, 34090 Montpellier (FR)
(72) Inventor: DALLEMAGNE, Patrick, 14260 SEULLINE (FR); ROCHAIS, Christophe, 14000 CAEN (FR); CLAEYSEN, Sylvie, 34980 SAINT-GELY-DU-FESC (FR)
(74) Representative: Lavoix

(57) **Abstract**

The present invention concerns a pharmaceutical composition comprising an acetylcholinesterase inhibitor and a 5-HT₄ receptor agonist, for use as neuroprotective agent in the prevention and/or treatment of a neurodegenerative disease. It also concerns a neuroprotective pharmaceutical combination comprising an acetylcholinesterase inhibitor and a 5-HT₄ receptor agonist for simultaneous, separate or sequential use as neuroprotective agent in the prevention and/or treatment of a neurodegenerative disease.

## Description

The present invention concerns the treatment of neurodegenerative diseases.

Neurodegenerative diseases are those which are induced by the collapse of nervous circuit neural network based on the systematic degeneration and deciduation of neurocytes, and they have been known as various intractable diseases such as Alzheimer's disease (AD), Parkinson's disease, Lewis body dementia, vascular dementia, and problem associated with ageing including (i) non-cognitive neurodegeneration, (ii) non-cognitive neuromuscular degeneration and (iii) motor-sensory neurodegeneration, Huntington's disease, motor neurone diseases including amyotrophic lateral sclerosis (ALS), Down syndrome, corticobasal ganglionic degeneration, multiple system atrophy, cerebral atrophy, olivopontocerebellar atrophy, supranuclear palsy, Friedreich's ataxia, spinocerebellar ataxia type 2, frontotemporal degenerations (FTD) and primary progressive aphasia

Clinical symptoms of neurodegenerative diseases vary from minor ones to severe ones, depending on the disease. They include tremor, rigidity, akinesia, hypokinesia, bradykinesia, attitude reflex failure, dysautonomia, pulsion, gait disturbance, depression, apathy, confusion, learning alteration, memory deficits, cognitive impairment, amyotrophia, muscle loss, disorder of shoulder girdle, articulation disorder, dysphagia, respiration disorder, numbness, paralysis and dementia, which are major hurdles in performing daily activities.

Neurodegenerative death is a complicated mechanism probably due to multiple molecular groups which trigger neurodegenerative diseases when issues in their expression, activity and/or regulation occur. This may explain why no effective method for inhibiting or reversing neurodegeneration has been established so far.

In addition to the treatment for removing causatives of such diseases, it is also important to reconstruct the neural network. For example, there have been said that, in Alzheimer's disease for which cytotoxicity of amyloid β peptide has been recognized as a causative, both the atrophia of neurites and the reduction of synapses trigger off the deterioration of neurofunction, and in reverse, even after such triggering, neurocytes which are not fully denatured or survived without degeneration can be recovered if they can possibly be activated to extend neurites for recovering synapse.

There is thus an important need for new agents capable of protecting neurites but also able to increase neurite growth and to promote the formation of new synapses, in order both to prevent and curatively treat neurodegenerative diseases.

The present invention meets this need.

The present invention indeed results from the unexpected finding by the inventors that a combination of acetylcholinesterase inhibitor activity and of 5-HT₄ receptor agonist activity has a neuroprotective activity since it is able, in addition to decrease Tau hyperphosphorylation, to increase neurite growth, to maintain the synapses rate, and most importantly and unexpectedly to promote formation of new synapses, which is not the case of a compound only displaying acetylcholinesterase inhibitor activity, such as Donepezil.

Because of this general and efficient protective effect on neurites, the use of a combination of acetylcholinesterase inhibitor activity and of 5-HT₄ receptor agonist activity is very promising as neuroprotective agent for preventing and/or treating any neurodegenerative disease involving neurites degeneration.

The present invention thus concerns a pharmaceutical composition comprising an acetylcholinesterase inhibitor and a 5-HT₄ receptor agonist for use as neuroprotective agent in the prevention and/or treatment of a neurodegenerative disease.

The present invention also concerns a neuroprotective pharmaceutical combination comprising an acetylcholinesterase inhibitor and a 5-HT₄ receptor agonist for simultaneous, separate or sequential use as neuroprotective agent in the prevention and/or treatment of a neurodegenerative disease.

The present invention also concerns an acetylcholinesterase inhibitor for use in combination with a 5-HT₄ receptor agonist as neuroprotective agent in the prevention and/or treatment of a neurodegenerative disease.

Another object of the invention concerns a 5-HT₄ receptor agonist for use in combination with an acetylcholinesterase inhibitor as neuroprotective agent in the prevention and/or treatment of a neurodegenerative disease.

### Detailed description of the invention

### Acetylcholinesterase inhibitor

By "acetylcholinesterase" or "AChE" is meant herein a hydrolase of the EC 3.1.1.7 group, which catalyzes the breakdown of acetylcholine and of some other choline esters that function as neurotransmitters.

By "acetylcholinesterase inhibitor" is meant herein any natural or synthesized compounds or molecules that influence the acetylcholine hydrolysis function of acetylcholinesterase.

In the present invention, the term "inhibit" acetylcholinesterase refers to reduce, decrease, eliminate, diminish, slow or stop the acetylcholine hydrolysis reaction of acetylcholinesterases.

Techniques to identify acetylcholinesterase inhibitors are well-known from the skilled person. Typically, acetylcholinesterase inhibitors can be identified using the Ellman's method disclosed in Ellman et al. (1961) Biochemical Pharmacology 7:88-95.

Examples of acetylcholinesterase inhibitors are well-known from the skilled person and include Donepezil of the following formula (I) : 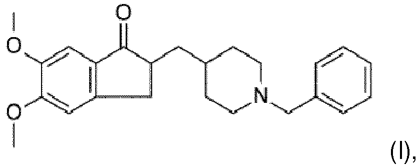 rivastigmine, tacrine, galantamine, the compounds disclosed in the US patents 6,037,352, US 6,037,327, US 6,022,683, US 5,935,781, US 5,886,051, US 5,869,484 (phenylcarbamate derivatives suitable as acetylcholinesterase inhibitors), US 5,798,392, US 5,789,425 (imidazolidinone derivatives), US 5,777,108 (galantamine derivatives as acetylcholinesterase inhibitors), US 5,731,284, US 5,589,386, US 5,585,375, US 5,550,253, US 5,547,960, US 5,541,340 and US 5,500,188.

In a particular embodiment, the acetylcholinesterase inhibitor is selected from the group consisting in Donepezil, rivastigmine, tacrine and galantamine.

In a particular embodiment, the acetylcholinesterase inhibitor is Donepezil.

### 5-HT₄ receptor agonist

By "5-HT₄ receptor" or "5-hydroxytryptamine receptor 4" is meant herein a protein encoded in humans by the *HTR4* gene and which is a member of the family of human serotonin receptors that stimulate cAMP production in response to serotonin (5-hydroxytryptamine).

By "5-HT₄ receptor agonist" is meant herein any natural or synthesized compounds or molecules that has an affinity for serotonin type-4 receptors and is able to mimic the stimulating effects of serotonin at this specific cellular receptor.

Techniques to identify 5-HT₄ receptor agonists are well-known from the skilled person. Typically, 5-HT₄ receptor agonists can be identified using the method first disclosed in Dumuis et al. (1988) Mol Pharmacol. 34:880-7 and updated in Gaven et al. (2013) Brain Res. 1511:65-72.

Examples of 5-HT₄ receptor agonists are well-known from the skilled person and include prucalopride, cisapride, dazopride, mosapride, renzapride, naronapride, zacopride, velusetrag, tegaserod, metoclopramide, cinitapride, YM-53389 {(+)-(S)-2-chloro-5-methoxy-4-[5-(2-piperidylmethyl)-1,2,4-oxadiazol-3-yl]aniline monohydrochloride}, RS-67333, 5-Methoxytryptamine (5-MT), RS-56532, ML-10302, SSP-002392, BIMU-8 and DA-6886 (*N*-((1-(3-(1,2,3-triazol-1-yl)propyl)piperidin-4-yl)methyl)-4-amino-5-chloro-2-methoxybenzamide hydrochloride).

In a particular embodiment, the 5-HT₄ receptor agonist is selected from the group consisting in prucalopride, cisapride, dazopride, mosapride, renzapride, naronapride, zacopride, velusetrag tegaserod, metoclopramide, cinitapride, YM-53389, RS-67333, 5-Methoxytryptamine (5-MT), RS-56532, ML-10302, SSP-002392, BIMU-8 and DA-6886.

In a particular embodiment, the 5-HT₄ receptor agonist is RS-67333.

In a particular embodiment, the acetylcholinesterase inhibitor and the 5-HT₄ receptor agonist are a unique compound which bears both activities.

Accordingly, in a particular embodiment, the acetylcholinesterase inhibitor and the 5-HT₄ receptor agonist are the donecopride compound of following formula (II):

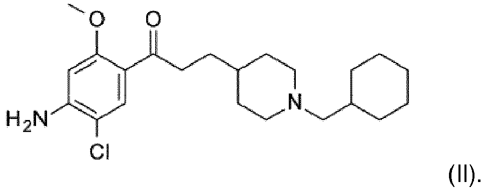

### Neurodegenerative disease

By "neurodegenerative disease" is meant herein a disease induced by the collapse of nervous circuit neural network based on the systematic degeneration and deciduation of neurocytes.

As explained above, the present inventors demonstrated that a combination of acetylcholinesterase inhibitory activity and of 5-HT₄ receptor agonist activity displayed a general and efficient protective effect on neurites. Because of this general neuroprotective effect, this combination can be used as neuroprotective agent for preventing and/or treating any neurodegenerative disease.

Examples of neurodegenerative disease include Alzheimer's disease (AD), Parkinson's disease, Lewis body dementia, vascular dementia, and problem associated with ageing including (i) non-cognitive neurodegeneration, (ii) non-cognitive neuromuscular degeneration and (iii) motor-sensory neurodegeneration, Huntington's disease, motor neurone diseases including amyotrophic lateral sclerosis (ALS), Down syndrome, corticobasal ganglionic degeneration, multiple system atrophy, cerebral atrophy, olivopontocerebellar atrophy, supranuclear palsy, Friedreich's ataxia, spinocerebellar ataxia type 2, frontotemporal degenerations (FTD) and primary progressive aphasia.

In a particular embodiment, the neurodegenerative disease is selected from the group consisting of Alzheimer's disease, Parkinson's disease, Huntington's disease, multiple system atrophy, motor neurone diseases including amyotrophic lateral sclerosis, Down syndrome and frontotemporal degenerations.

In another embodiment, the neurodegenerative disease is Alzheimer's disease.

In an alternative embodiment, the neurodegenerative disease is a neurodegenerative disease which is not Alzheimer's disease.

### Medical indications

The present invention relates to a pharmaceutical composition comprising an acetylcholinesterase inhibitor as defined in the section "*Acetylcholinesterase inhibitor*" above and a 5-HT₄ receptor agonist as defined in the section "*5-HT₄ receptor agonist*" above for use as neuroprotective agent in the prevention and/or treatment of a neurodegenerative disease as defined in the section "*Neurodegenerative disease*" above.

The present invention also concerns the use of a pharmaceutical composition comprising an acetylcholinesterase inhibitor as defined in the section "*Acetylcholinesterase inhibitor*" above and a 5-HT₄ receptor agonist as defined in the section "*5-HT₄ receptor agonist*" above for the manufacture of a neuroprotective agent for the prevention and/or treatment of a neurodegenerative disease as defined in the section "*Neurodegenerative disease*" above.

The present invention further concerns a method of preventing and/or treating a neurodegenerative disease as defined in the section "*Neurodegenerative disease*" above, comprising administering to a subject in need thereof a therapeutically efficient amount of a pharmaceutical composition comprising an acetylcholinesterase inhibitor as defined in the section "*Acetylcholinesterase inhibitor*" above and a 5-HT₄ receptor agonist as defined in the section "*5-HT₄ receptor agonist*" above as neuroprotective agent.

The present invention further concerns a method of neuroprotection in a subject suffering from, or at risk of suffering from a neurodegenerative disease as defined in the section "*Neurodegenerative disease*" above, comprising administering to said subject a therapeutically efficient amount of a pharmaceutical composition comprising an acetylcholinesterase inhibitor as defined in the section *"Acetylcholinesterase inhibitor"* above and a 5-HT₄ receptor agonist as defined in the section "*5-HT₄ receptor agonist*" above.

The present invention also concerns a neuroprotective pharmaceutical combination comprising an acetylcholinesterase inhibitor as defined in the section *"Acetylcholinesterase inhibitor"* above and a 5-HT₄ receptor agonist as defined in the section "*5-HT₄ receptor agonist*" above for simultaneous, separate or sequential use as neuroprotective agent in the prevention and/or treatment of a neurodegenerative disease as defined in the section *"Neurodegenerative disease"* above.

The present invention also concerns the use of an acetylcholinesterase inhibitor as defined in the section *"Acetylcholinesterase inhibitor"* above and a 5-HT₄ receptor agonist as defined in the section "*5-HT₄ receptor agonist*" above for the manufacture of a neuroprotective pharmaceutical combination for simultaneous, separate or sequential administration for the prevention and/or treatment of a neurodegenerative disease as defined in the section *"Neurodegenerative disease"* above.

The present invention also concerns a method of preventing and/or treating a neurodegenerative disease as defined in the section *"Neurodegenerative disease"* above, comprising simultaneously, separately or sequentially administering to a subject in need thereof a therapeutically efficient amount of an acetylcholinesterase inhibitor as defined in the section *"Acetylcholinesterase inhibitor"* above and a 5-HT₄ receptor agonist as defined in the section "*5-HT₄ receptor agonist*" above as neuroprotective agent.

The present invention also concerns a method of neuroprotection in a subject suffering from, or at risk of suffering from a neurodegenerative disease as defined in the section *"Neurodegenerative disease"* above, comprising simultaneously, separately or sequentially administering to said subject a therapeutically efficient amount of an acetylcholinesterase inhibitor as defined in the section *"Acetylcholinesterase inhibitor"* above and a 5-HT₄ receptor agonist as defined in the section "*5-HT₄ receptor agonist*" above.

According to the invention, the term "subject" or "subject in need thereof" is intended for a human or non-human mammal affected or likely to be affected with a neurodegenerative disease as defined in the section "*Neurodegenerative diseases*" above.

In the context of the invention, the term "treating" or "treatment" means reversing, alleviating or inhibiting the progress of the disorder or condition to which such term applies, or one or more symptoms of such disorder or condition.

In the context of the invention, the term "preventing" or "prevention" means delaying or preventing the onset of the disorder or condition to which such term applies, or one or more symptoms of such disorder or condition.

By "neuroprotection" is meant herein the prevention or inhibition of degenerative effects and restauration of neuronal integrity in the central nervous system, in particular the prevention or inhibition of neuronal degeneration.

By "neuroprotective agent" is meant herein a combination of neuroprotection as defined above.

The inventors more particularly demonstrated that the combination of compounds used in the context of the invention was capable of increasing neurite growth, maintaining synapses rate, decreasing Tau hyperphosphorylation and/or promoting formation of new synapses.

Accordingly, in a particular embodiment, the pharmaceutical composition comprising an acetylcholinesterase inhibitor as defined in the section *"Acetylcholinesterase inhibitor*" above and a 5-HT₄ receptor agonist as defined in the section "*5-HT₄ receptor agonist*" above, or the neuroprotective pharmaceutical combination comprising an acetylcholinesterase inhibitor as defined in the section *"Acetylcholinesterase inhibitor*" above and a 5-HT₄ receptor agonist as defined in the section "*5-HT₄ receptor agonist*" above, is for increasing neurite growth.

The present invention also concerns a method for increasing neurite growth in a subject suffering from, or at risk of suffering from a neurodegenerative disease as defined in the section *"Neurodegenerative disease"* above, comprising
- administering to said subject a therapeutically efficient amount of a pharmaceutical composition comprising an acetylcholinesterase inhibitor as defined in the section *"Acetylcholinesterase inhibitor*" above and a 5-HT₄ receptor agonist as defined in the section "*5-HT₄ receptor agonist"* above, or
- simultaneously, separately or sequentially administering to said subject a therapeutically efficient amount of an acetylcholinesterase inhibitor as defined in the section *"Acetylcholinesterase inhibitor"* above and a 5-HT₄ receptor agonist as defined in the section "*5-HT₄ receptor agonist*" above.

By "neurite growth" is meant herein the extension process of any projection from the cell body of a neuron and associated synapse genesis.

By "increasing neurite growth" is meant herein that the growth of neurites is higher (*i*.*e*. more efficient, faster or longer), preferably statistically significantly higher, when the compounds of interest are applied compared to the growth of neurites observed in the same conditions but without applying the compounds of interest. Preferably, the neurite growth is increased of at least 120% compared to growth of neurites observed in the same conditions but without applying the compound of interest.

In another particular embodiment, the pharmaceutical composition comprising an acetylcholinesterase inhibitor as defined in the section *"Acetylcholinesterase inhibitor"* above and a 5-HT₄ receptor agonist as defined in the section "*5-HT₄ receptor agonist*" above, or the neuroprotective pharmaceutical combination comprising an acetylcholinesterase inhibitor as defined in the section *"Acetylcholinesterase inhibitor"* above and a 5-HT₄ receptor agonist as defined in the section "*5-HT₄ receptor agonist*" above, is for maintaining synapses rate.

Another object of the present invention concerns a method for maintaining synapses rate in a subject suffering from, or at risk of suffering from a neurodegenerative disease as defined in the section *"Neurodegenerative disease"* above, comprising:
- administering to said subject a therapeutically efficient amount of a pharmaceutical composition comprising an acetylcholinesterase inhibitor as defined in the section *"Acetylcholinesterase inhibitor*" above and a 5-HT₄ receptor agonist as defined in the section "*5-HT₄ receptor agonist*" above, or
- simultaneously, separately or sequentially administering to said subject a therapeutically efficient amount of an acetylcholinesterase inhibitor as defined in the section *"Acetylcholinesterase inhibitor*" above and a 5-HT₄ receptor agonist as defined in the section "*5-HT₄ receptor agonist*" above.

By "synapses rate" is meant herein the ratio of the number of synapses and the number of neurons.

By "maintaining synapses rate" is meant herein that the synapses rate is similar (*i*.*e*. not statistically significantly different) when the compounds of interest are applied compared to the synapses rate observed in control conditions, such as same conditions but without applying the compounds of interest or conditions where the neurons are not faced to neurodegenerative conditions.

In another particular embodiment, the pharmaceutical composition comprising an acetylcholinesterase inhibitor as defined in the section "*Acetylcholinesterase inhibitor*" above and a 5-HT₄ receptor agonist as defined in the section "*5-HT₄ receptor agonist*" above, or the neuroprotective pharmaceutical combination comprising an acetylcholinesterase inhibitor as defined in the section "*Acetylcholinesterase inhibitor*" above and a 5-HT₄ receptor agonist as defined in the section "*5-HT₄ receptor agonist*" above, is for promoting formation of new synapses.

Another object of the present invention concerns a method for promoting formation of new synapses in a subject suffering from, or at risk of suffering from a neurodegenerative disease as defined in the section *"Neurodegenerative disease"* above, comprising:
- administering to said subject a therapeutically efficient amount of a pharmaceutical composition comprising an acetylcholinesterase inhibitor as defined in the section *"Acetylcholinesterase inhibitor*" above and a 5-HT₄ receptor agonist as defined in the section "*5-HT₄ receptor agonist*" above, or
- simultaneously, separately or sequentially administering to said subject a therapeutically efficient amount of an acetylcholinesterase inhibitor as defined in the section *"Acetylcholinesterase inhibitor*" above and a 5-HT₄ receptor agonist as defined in the section "*5-HT₄ receptor agonist*" above.

By "promoting formation of new synapses" is meant herein that the number of newly formed synapses is higher, preferably statistically significantly higher, when the compounds of interest are applied compared to the number of newly formed synapses observed in the same conditions but without applying the compounds of interest. Preferably, the formation of new synapses is increased of at least 120% compared to formation of new synapses observed in the same conditions but without applying the compound of interest.

In another particular embodiment, the pharmaceutical composition comprising an acetylcholinesterase inhibitor as defined in the section *"Acetylcholinesterase inhibitor"* above and a 5-HT₄ receptor agonist as defined in the section "*5-HT₄ receptor agonist*" above, or the neuroprotective pharmaceutical combination comprising an acetylcholinesterase inhibitor as defined in the section *"Acetylcholinesterase inhibitor*" above and a 5-HT₄ receptor agonist as defined in the section "*5-HT₄ receptor agonist*" above, is for decreasing Tau hyperphosphorylation.

Another object of the present invention concerns a method for decreasing Tau hyperphosphorylation in a subject suffering from, or at risk of suffering from a neurodegenerative disease as defined in the section *"Neurodegenerative disease"* above, comprising:
- administering to said subject a therapeutically efficient amount of a pharmaceutical composition comprising an acetylcholinesterase inhibitor as defined in the section *"Acetylcholinesterase inhibitor*" above and a 5-HT₄ receptor agonist as defined in the section "*5-HT₄ receptor agonist*" above, or
- simultaneously, separately or sequentially administering to said subject a therapeutically efficient amount of an acetylcholinesterase inhibitor as defined in the section *"Acetylcholinesterase inhibitor*" above and a 5-HT₄ receptor agonist as defined in the section "*5-HT₄ receptor agonist*" above.

By "Tau hyperphosphorylation" is meant herein the abnormally high phosphorylation of the Tau protein, in particular the abnormal phosphorylation of the Tau protein at least one of the Thr39, Ser46, Thr50, Ser68, Thr69, Thr71, Ser113, Thr153, Thr175, Thr181, Ser184, Ser185, Ser191, Ser198, Ser199, Ser202, Thr205, Ser208, Ser210, Thr212, Ser214, Thr217, Thr231, Ser235, Ser237, Ser238, Ser241, Ser258, Ser262, Ser285, Ser289, Ser305, Ser324, Ser352, Ser356, Tyr394, Ser396, Ser400, Thr403, Ser404, Ser409, Ser412, Ser413, Thr414, Ser416, Ser422, Ser433 and Ser435 residues.

By "decreasing Tau hyperphosphorylation" is meant herein that at least one of the Thr39, Ser46, Thr50, Ser68, Thr69, Thr71, Ser113, Thr153, Thr175, Thr181, Ser184, Ser185, Ser191, Ser198, Ser199, Ser202, Thr205, Ser208, Ser210, Thr212, Ser214, Thr217, Thr231, Ser235, Ser237, Ser238, Ser241, Ser258, Ser262, Ser285, Ser289, Ser305, Ser324, Ser352, Ser356, Tyr394, Ser396, Ser400, Thr403, Ser404, Ser409, Ser412, Ser413, Thr414, Ser416, Ser422, Ser433 and Ser435 residues, in particular one of Ser212 and Thr214 residues of the Tau protein is not abnormally phosphorylated when the compounds of interest are applied whereas said residue is abnormally phosphorylated in the same conditions but without applying the compounds of interest.

By "therapeutically effective amount" is meant herein sufficient amount of the pharmaceutical composition or combination to act as neuroprotective agent in the treatment and/or prevention of a neurodegenerative disease at a reasonable benefit/risk ratio applicable to any medical treatment. It will be understood, however, that the total daily usage of the compounds of the present invention will be decided by the attending physician within the scope of sound medical judgment. The specific therapeutically effective dose level for any particular subject will depend upon a variety of factors including the disorder being treating and the severity of the disorder, activity of the specific compounds employed, the age, body weight, general health, sex and diet of the subject, the time of administration, route of administration and rate of excretion of the specific compounds employed, the duration of the treatment, drugs used in combination or coincidental with the specific compounds employed, and like factors well known in the medical arts. For example, it is well within the skill of the art to start doses of the compounds at levels lower than those required to achieve the desired therapeutic effect and to gradually increase the dosage until the desired effect is achieved.

As used herein, the term "combination", "therapeutic combination" or "pharmaceutical combination", defines either a fixed combination in one dosage unit form or a kit of parts for the combined administration where the acetylcholinesterase inhibitor and the 5-HT₄ receptor agonist may be administered independently at the same time or separately within time intervals that allow that the combination partners show a synergistic effect.

The compounds of the combination of the invention can thus be formulated in one or two separate pharmaceutical compositions, each composition being for the same or different administration routes.

The compounds used in the context of the invention may be administered in the form of a pharmaceutical composition including pharmaceutically acceptable excipients, and optionally sustained-release matrices, such as biodegradable polymers, to form therapeutic compositions.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

The form of the pharmaceutical compositions including the compounds used in the context of the invention and the route of administration naturally depend upon the condition to be treated, the severity of the illness, the age, weight, and gender of the patient, etc.

The compounds used in the context of the invention can be formulated for a topical, oral, parenteral, intranasal, intravenous, intramuscular, subcutaneous or intraocular administration and the like. In a particular embodiment, the compounds used in the context of the invention are administered orally.

Alternatively, the pharmaceutical compositions including the compounds used in the context of the invention may contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

To prepare pharmaceutical compositions, an effective amount of the compounds used in the context of the invention may be dissolved or dispersed in a pharmaceutically acceptable carrier or aqueous medium.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

The carrier can be a solvent or dispersion medium containing, for example, water, ethanol, polyol (for example, glycerol, propylene glycol, and liquid polyethylene glycol, and the like) and suitable mixtures thereof. The proper fluidity can be maintained, for example, by the use of a coating, such as lecithin, by the maintenance of the required particle size in the case of dispersion and by the use of surfactants, stabilizing agents, cryoprotectants or antioxidants. The prevention of the action of microorganisms can be brought about by antibacterial and antifungal agents. In many cases, it will be preferable to include isotonic agents, for example, sugars or sodium chloride.

Sterile injectable solutions are prepared by incorporating the active compounds in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 mL of isotonic NaCl solution and either added to 1000 mL of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

The compounds used in the context of the invention can typically be administered orally for example in the form of tablets, capsules, microcapsules, powders, granules, syrups, solutions or suspensions taken by the oral or sublingual route.

In a particular embodiment, the compounds used in the context of the invention are suitably administered orally at a daily dose of 1 to 20 mg/kg of body weight, in particular at a daily dose of 2 mg/kg.

In another particular embodiment, the compounds used in the context of the invention are suitably administered orally, twice a week, at a dose of 1 to 20 mg/kg of body weight, in particular at a dose of 1 mg/kg twice a week.

According to another particular embodiment, the compounds used in the context of the invention are administered at doses of 10 to 1000 mg per day, preferably at doses of 0.2 to 2 mg 5 times a day.

The present invention will be further illustrated by the examples below.

### Examples

### Example 1

This example shows the effect of a compound bearing both the acetylcholinesterase inhibitory activity and the 5-HT₄ receptor agonist activity (namely Donecopride) in rat primary hippocampal neurons injured with Aβ (24h exposure).

Alzheimer disease (AD) is a neurodegenerative disease which affects mainly people over the age of 65, suffering from different clinical symptoms such as progressive decline in thinking, language, and learning capacity. Increasing evidence from various sources points to Aβ Oligomers (AβO)/protofibrils as putative toxic species in AD pathogenesis.

By generating a solution of AβO and playing on the concentration of and time of exposure to AβO, it was possible to reproduce early effects (oxidative stress) and the long-term development of structural alterations (death of neurons).

The model used in the present example, using Aβ peptide solution containing AβO (precisely measured by automatic Western Blot), reproduced essential neuropathological features of AD.

The aim of this study was to evaluate the effects of a compound bearing both acetylcholinesterase inhibitory activity and 5-HT₄ receptor agonist activity (Donecopride fumarate at several concentrations) in rat primary hippocampal neurons injured with Aβ (24h exposure). A compound bearing only an acetylcholinesterase inhibitory activity, namely Donepezil, (one concentration) was used as reference test compound.

### Materials and methods

### Primary culture of hippocampal neurons

Rat hippocampal neurons were cultured as described by Callizot et al. (2013) J. Neurosci. Res. 91706-716. Pregnant female rats of 17 days gestation (Rats Wistar; Janvier Labs France) were killed using a deep anesthesia with CO₂ chamber followed by a cervical dislocation. Then, fetuses were removed from the uterus and immediately placed in ice-cold L15 Leibovitz medium with a 2% penicillin (10,000 U/ml) and streptomycin (10 mg/ml) solution (PS) and 1% bovine serum albumin (BSA). Hippocampal neurons were treated for 20 min at 37°C with a trypsin-EDTA solution at a final concentration of 0.05% trypsin and 0.02% EDTA. The dissociation was stopped by adding Dulbecco's modified Eagle's medium (DMEM) with 4.5 g/l of glucose, containing DNAse I grade II (final concentration 0.5 mg/ml) and 10% fetal calf serum (FCS). Cells were mechanically dissociated by three forced passages through the tip of a 10-ml pipette and then centrifuged at 515 x g for 10 min at 4°C. The supernatant was discarded, and the pellet was resuspended in a defined culture medium consisting of Neurobasal medium with a 2% solution of B27 supplement, 2 mmol/I of L-glutamine, 2% of PS solution, and 10 ng/ml of brain-derived neurotrophic factor (BDNF). Viable cells were counted in a Neubauer cytometer, using the trypan blue exclusion test. Cells were seeded at a density of 20,000 per well in 96-well plates precoated with poly-L-lysine and cultured at 37°C in an air (95%)-CO₂ (5%) incubator. The medium was changed every 2 days.

### Donecopride and human Aβ1-42 exposure

The hippocampal neurons were exposed with Aβ solutions (see below) after 17 days of culture. The Aβ1-42 preparation was done following the procedure described by Callizot et al. (2013) J. Neurosci. Res. 91706-716. Briefly, Aβ1-42 peptide was dissolved in the defined culture medium mentioned above, at an initial concentration of 40 µM. This solution was gently agitated for 3 days at 37°C in the dark and immediately used after being properly diluted in culture medium to the concentrations used (20 µM (plate 1) or 2.5 µM (plate 2) corresponding to 2 µM or 0.25 µM of oligomers (AβO) respectively).

Donecopride fumarate was solved in culture medium and preincubated 1 h before Aβ application. Aβ1-42 preparation was added to a final concentration of 20 or 2.5 µM (= 2 µM or 0.25 µM of AβO, evaluated by automatic Western Blot) diluted in control medium in presence of Donecopride.

### Organization of culture plates

Donecopride fumarate (Donecopride) was tested on one culture in a 96 well plate (6 wells per conditions). The compound was preincubated one hour before Aβ application. The following conditions were assessed:

| PLATE 1 (MAP-2 / Tau) | PLATE 2 (PSD95 / SYN) |
|---|---|
| Control (Vehicle) | Control (Vehicle) |
| + Aβ (20 µM 24 h) / vehicle | + Aβ (2.5 µM 24 h) / vehicle |
| + Aβ (20 µM 24 h) / Donecopride (1 µM) | + Aβ (2.5 µM 24 h) / Donecopride (1 µM) |
| + Aβ (20 µM 24 h) / Donecopride (500 nM) | + Aβ (2.5 µM 24 h) / Donecopride (500 nM) |
| + Aβ (20 µM 24 h) / Donecopride (100 nM) | + Aβ (2.5 µM 24 h) / Donecopride (100 nM) |
| + Aβ (20 µM 24 h) / Donecopride (50 nM) | + Aβ (2.5 µM 24 h) / Donecopride (50 nM) |
| + Aβ (20 µM 24 h) / Donecopride (10 nM) | + Aβ (2.5 µM 24 h) / Donecopride (10 nM) |
| + Aβ (20 µM 24 h) / Donecopride (5 nM) | + Aβ (2.5 µM 24 h) / Donecopride (5 nM) |
| + Aβ (20 µM 24 h) / Donecopride (1 nM) | + Aβ (2.5 µM 24 h) / Donecopride (1 nM) |
| + Aβ (20 µM 24 h) / Donepezil (1 µM) | + Aβ (2.5 µM 24 h) / Donepezil (1 µM) |

### End point evaluation

### Plate 1: SURVIVAL, NEURITE NETWORK AND PHOSPHO TAU EVALUATION

24 hours after intoxication, the hippocampal neurons were fixed by a cold solution of ethanol (95%) and acetic acid (5%) for 5 min at -20°C. After permeabilization with 0.1% of saponin, cells were incubated for 2 h with:
a) chicken polyclonal antibody anti microtubule-associated-protein 2 (MAP-2), diluted at 1/1000 in PBS containing 1% fetal calf serum and 0.1% of saponin (this antibody allows the specific staining of neuronal cell bodies and neurites; and allow the study of neuronal cell death and neurite network).
b) mouse monoclonal antibody anti-phospho Tau (AT100) at dilution of 1/400 in PBS containing 1% fetal calf serum and 0.1% of saponin.

These antibodies were revealed with Alexa Fluor 488 goat anti mouse IgG and Alexa Fluor 568 goat anti-chicken IgG at the dilution 1/400 in PBS containing 1% FCS, 0.1% saponin, for 1 h at room temperature.

For each condition, 30 pictures (representative of the whole well area) per well were taken using ImageXpress (Molecular Devices) at 20x magnification. All images were taken with the same acquisition parameters. Analyses were performed automatically by using Custom Module Editor (Molecular Devices).

The following endpoints were assessed:
- Total number of neurons (neuron survival, number of MAP-2 positive neurons)
- Neurite network (in µm of MAP-2 positive neurons)
- area of tau in neurons (µm², overlapping with MAP-2 positive neurons).

### Plate 2: SYNAPSES EVALUATION

24 hours after intoxication, the hippocampal neurons were fixed by a cold solution of ethanol (95%) and acetic acid (5%) for 5 min at -20°C. After permeabilization with 0.1% of saponin, cells were incubated for 2 h with:
a) mouse monoclonal antibody anti post synaptic density 95kDa (PSD95) at a dilution of 1/100 in PBS containing 1% fetal calf serum and 0.1% of saponin.
b) Rabbit polyclonal antibody anti-synaptophysin (SYN) at the dilution of 1/100 in PBS containing 1% fetal calf serum and 0.1% of saponin.

These antibodies were revealed with Alexa Fluor 488 goat anti mouse IgG and Alexa Fluor 568 goat anti-rabbit IgG at the dilution 1/400 in PBS containing 1% FCS, 0.1% saponin, for 1 h at room temperature.

For each condition, 40 pictures per well were taken using ImageXpress (Molecular Devices) with 40x magnification. All images were taken with the same acquisition parameters.

Synapses evaluation was performed automatically by using Custom module editor (Molecular Devices).

The following endpoints were assessed:
- Total number of synapses (overlapping between PSD95/SYN).

### Statistical analysis

All values are expressed as mean +/- SEM. Statistical analysis was performed by one-way ANOVA, followed by PLSD Fisher's test, p < 0.05 were considered significant.

Graphs and statistical analyses on the different conditions were performed using GraphPad Prism software version 7.04. *p<0.05 was considered significant.

### Results

### Effects of Donecopride on the survival and neurite network of MAP-2 hippocampal neurons and on the hyperphosphorylation of Tau.

### Neuronal survival

The effect of Donecopride on the survival of MAP-2 neurons in a primary culture of hippocampal neurons is shown in Table 1 below.

**Table 1: MAP-2 neurons (% of control)**

| | **MAP-2 neurons (% of control)** | | |
|---|---|---|---|
| | **mean** | **sem** | **N** |
| Control | 100 | 5 | 6 |
| + Aβ 1-42 (20 µM) | 70 | 2 | 6 |
| + Donecopride (1 nM) | 70 | 2 | 5 |
| + Donecopride (5 nM) | 69 | 2 | 6 |
| + Donecopride (10 nM) | 73 | 2 | 5 |
| + Donecopride (50 nM) | 74 | 2 | 5 |
| + Donecopride (100 nM) | **78*** | 1 | 4 |
| + Donecopride (500 nM) | **79*** | 1 | 5 |
| + Donecopride (1 µM) | **62*** | 2 | 5 |
| + Donepezil (1 µM) | **82*** | 2 | 5 |

| | | | |
|---|---|---|---|
| * : p < 0.05 versus Aβ1-42, determined by one-way ANOVA followed by a Fisher's LSD test. | | | |

An important loss of MAP-2 neurons was consecutive to the application of the Aβ peptide. Donepezil, used here as a control of acetylcholinesterase inhibitory activity alone, was able to significantly protect neurons from death. Two doses of Donecopride (100 nM and 500 nM) exerted neuroprotective effects to a similar extent to Donepezil.

### Neurite network

The effect of Donecopride on the neurite network of MAP-2 neurons in a primary culture of hippocampal neurons is shown in Table 2 below.

**Table 2: MAP-2 neurite network (% of control)**

| | **MAP-2 neurite network (% of control)** | | |
|---|---|---|---|
| | **mean** | **sem** | **N** |
| Control | 100 | 2 | 6 |
| + Aβ 1-42 (20 µM) | 63 | 1 | 6 |
| + Donecopride (1 nM) | 64 | 2 | 5 |
| + Donecopride (5 nM) | 69 | 2 | 6 |
| + Donecopride (10 nM) | 68 | 2 | 5 |
| + Donecopride (50 nM) | **71*** | 3 | 5 |
| + Donecopride (100 nM) | **73*** | 1 | 5 |
| + Donecopride (500 nM) | **79*** | 3 | 6 |
| + Donecopride (1 µM) | **76*** | 2 | 6 |
| + Donepezil (1 µM) | **92*** | 3 | 5 |

| | | | |
|---|---|---|---|
| * : p < 0.05 versus Aβ1-42, determined by one-way ANOVA followed by a Fisher's LSD test. | | | |

Total neurite network was severely shrunk upon Aβ 1-42 injury. Donepezil has significantly preserved almost all the neurite network. Four doses of Donecopride (50 nM, 100 nM, 500 nM and 1µM) exerted also protective effects on the neurite network.

The neurite network is proportional to the number of neurons in the culture. The total neurite network was normalized by the number of neurons, in order to determine the average length of neurite per neurons. The corresponding results are shown in Table 3 below.

**Table 3: Neurite network / MAP-2 neurons (% of control)**

| | **Neurite network / MAP-2 neurons (% of control)** | | |
|---|---|---|---|
| | **mean** | **sem** | **N** |
| Control | 100 | 6 | 6 |
| + Aβ 1-42 (20 µM) | 90 | 3 | 6 |
| + Donecopride (1 nM) | 90 | 3 | 4 |
| + Donecopride (5 nM) | 99 | 2 | 6 |
| + Donecopride (10 nM) | 96 | 3 | 4 |
| + Donecopride (50 nM) | 91 | 5 | 4 |
| + Donecopride (100 nM) | 93 | 1 | 4 |
| + Donecopride (500 nM) | 100 | 4 | 5 |
| + Donecopride (1 µM) | **124*** | 5 | 5 |
| + Donepezil (1 µM) | **113*** | 4 | 4 |

| | | | |
|---|---|---|---|
| * : p < 0.05 versus Aβ1-42, determined by one-way ANOVA followed by a Fisher's LSD test. | | | |

This adjustment showed that neurite length per neuron was longer in presence of Donepezil and Donecopride (1µM), suggesting that these two compounds had an effect on neurite outgrowth.

### Tau phosphorylation

The effect of Donecopride on the phosphorylation of Tau (AT100) in MAP-2 neurons in a primary culture of hippocampal neurons is shown in Table 4 below.

**Table 4: Area of Tau (AT100)/neurons (% of control)**

| | **Area of Tau (AT100)/neurons (% of control)** | | |
|---|---|---|---|
| | **mean** | **sem** | **N** |
| Control | 100 | 4 | 5 |
| + Aβ 1-42 (20 µM) | 200 | 11 | 5 |
| + Donecopride (1 nM) | 204 | 7 | 4 |
| + Donecopride (5 nM) | 201 | 8 | 5 |
| + Donecopride (10 nM) | 175 | 11 | 4 |
| + Donecopride (50 nM) | **161*** | 12 | 5 |
| + Donecopride (100 nM) | **154*** | 6 | 3 |
| + Donecopride (500 nM) | **152*** | 8 | 4 |
| + Donecopride (1 µM) | 198 | 8 | 5 |
| + Donepezil (1 µM) | **151*** | 6 | 4 |

| | | | |
|---|---|---|---|
| * : p < 0.05 versus Aβ1-42, determined by one-way ANOVA followed by a Fisher's LSD test. | | | |

An hyperphosphorylation of Tau (on AT100) was observed under application of Aβ 1-42. Donepezil was able to significantly mitigate this hyperphosphorylation. Similarly, Donecopride (50 nM, 100 nM, 500 nM) has significantly reduced the hyperphosphorylation of Tau on a dose-dependent manner.

### Effects of Donecopride on synapses in a primary culture of hippocampal neurons

To assess the formation of synapses, the distribution of PSD-95, a post-synaptic marker, and synaptophysin, a pre-synaptic marker, was studied. A structure positive for both PSD-95 and synaptophysin staining was considered as a synapse.

The effect of Donecopride on these synapses is shown in Table 5 below.

**Table 5: Number of synapses (% of control)**

| | **Number of synapses (% of control)** | | |
|---|---|---|---|
| | **mean** | **sem** | **N** |
| Control | **100*** | 6 | 5 |
| + Aβ 1-42 (20 µM) | 67 | 4 | 6 |
| + Donecopride (1 nM) | 69 | 5 | 4 |
| + Donecopride (5 nM) | **83*** | 3 | 5 |
| + Donecopride (10 nM) | **87*** | 7 | 5 |
| + Donecopride (50 nM) | **91*** | 3 | 6 |
| + Donecopride (100 nM) | **93*** | 3 | 4 |
| + Donecopride (500 nM) | **94*** | 5 | 4 |
| + Donecopride (1 µM) | **85*** | 4 | 5 |
| + Donepezil (1 µM) | **88*** | 6 | 5 |

| | | | |
|---|---|---|---|
| * : p < 0.05 versus Aβ1-42, determined by one-way ANOVA followed by a Fisher's LSD test. | | | |

The stress resulting from the application of Aβ 1-42 caused a reduction in the number of synapses (as previously shown by Callizot et al. (2013) J. Neurosci. Res. 91:706-716).

Donepezil mitigated the loss of synapses. Similarly, Donecopride was also able to preserve the number of synapses, on a dose dependent manner (from 5 nM to 1 µM). Interestingly, this effect was observed a low dose of the compound (5 nM). This effect increased with the dose.

Due to the injury caused by the Aβ 1-42 peptide, the number of neurons was reduced in the experimental conditions, which led to a reduction in the number of synapses. Therefore, to estimate the number of synapses per neuron, the number of synapses was normalized by the number of neurons for the control, Aβ 1-42, Donecopride (500 nM, the condition showing the highest number of synapses), and Donepezil groups.

This adjustment showed that ONLY DONECOPRIDE significantly promoted the formation of new synapses while Donepezil had no effect.

### Conclusion

The present study indicates that a combination of acetylcholinesterase inhibitory activity and 5-HT₄ receptor agonist activity has beneficial effects on the neuronal survival and neurite network of hippocampal neurons injured with Aβ 1-42 peptide, an *in vitro* model of Alzheimer disease.

Moreover, the compound bearing this combination of activities was able to reduce the hyperphosphorylation of Tau on the site AT100 and was able to significantly stimulate the formation of new synapses.

The effects of this combination were greater than those of Donepezil, suggesting that this combination is an interesting drug candidate as neuroprotective agent, in particular for treating neurodegenerative diseases.

### Example 2

This example shows the anti-amnesic, anti-amyloid and anti-inflammatory effects of a compound bearing both an acetylcholinesterase inhibitory activity and a 5-HT₄ receptor agonist activity (Donecopride) in chronic administration to the 5XFAD mouse model of Alzheimer's disease.

### Study rationale

The objective of the study was to evaluate the potential anti-amnesic, anti-amyloid and anti-inflammatory effects of a compound bearing both an acetylcholinesterase inhibitory activity and a 5-HT₄ receptor agonist activity (Donecopride) in chronic administration in a mouse model of Alzheimer's disease.

The mouse model used was the 5XFAD strain (referred at the Jackson Laboratory as "B6SJL-Tg(APPSwFILon,PSEN1 *M146L*L286V)6799Vas/Mmjax", MMRRC Stock No: 34840-JAX or "B6.Cg-Tg(APPSwFILon,PSEN1*M146L*L286V)6799Vas/ Mmjax, MMRRC Stock No: 34848-JAX).

These 5XFAD transgenic mice overexpress mutant human APP(695) with the Swedish (K670N, M671L), Florida (I716V), and London (V717I) Familial Alzheimer's Disease (FAD) mutations along with human PS1 harboring two FAD mutations, M146L and L286V. Both transgenes are regulated by the mouse Thy1 promoter to drive overexpression in the brain. 5XFAD mice recapitulate major features of Alzheimer's Disease amyloid pathology and are a useful model of intraneuronal Aβ42 induced neurodegeneration and amyloid plaque formation.

### Methods and Experimental Design

### Mice

Animal experiments were carried out in accordance with the Directive by the Council of the European Communities of November 24, 1986 (86/609/EEC). All efforts were made to minimize animal suffering and to reduce the number of mice used. The generation of 5XFAD mice was described in Oakley et al. (2006) J. Neurosci. 26:10129-10140. These transgenic mice overexpress both human APP (695) harboring the Swedish (K670N, M671L), Florida (1716V) and London (V717I) familial AD (FAD) mutations and human Presenilin 1 (PS1) harboring the two FAD mutations M146L and L286V. Expression of both transgenes is regulated by neuronal-specific elements of the mouse *Thy1* promoter. The 5XFAD strain (B6/SJL genetic background) was maintained by crossing hemizygous transgenic mice with B6/SJL F1 breeders. The 5XFAD strain (C57BL/6J genetic background) was maintained by crossing hemizygous transgenic mice with C57BL/6J F1 breeders. All animals were genotyped by PCR using tail genomic DNA. Transgenic and WT mice were bred in our animal facility, had access to food and water ad *libitum* and were housed under a 12 h light-dark cycle at 22-24°C. Female 5XFAD mice were used in this study. For each experiment, the age of mice is indicated in months (with a two-week range).

### Drugs

Donecopride (Donecopride fumarate, MR 31155) was synthesized in the CERMN, Caen, France.

RS 67333 (1-(4-amino-5-chloro-2-methoxy-phenyl)-3-(1-butyl-4-piperidinyl)-1-propanone), was used as a reference 5-HT₄ receptor agonist and was purchased from Tocris Bioscience (R&D Systems Europe, Lille, France).

Compounds were resuspended in DMSO (37.5 µg/µl, stored at -20°C) and freshly diluted 1:250 in 0.9% NaCl prior administration. Vehicle solution (0.9% NaCl, 0.4% DMSO) was used as control.

### Animal treatments

5XFAD mice received chronic treatments with drugs or vehicle according to 2 different protocols. In each experiment, drugs or vehicle solution were administered i.p. twice a week (1 mg/kg). In "Protocol 1", mice (n = 6-7 mice per group) were treated with compounds for two months, from 2 to 4 months of age. In "Protocol 2", mice (n = 7) were treated with compounds for three months, from 1 month to 4 months of age. At the end of each protocol, mice were anesthetized with a mixture of 100 mg/kg ketamine and 10 mg/kg xylazine in saline solution and perfused transcardially with PBS. Brains were quickly isolated on ice, the olfactory bulbs and cerebellum removed and the two hemispheres divided. One hemisphere was frozen on dry ice and stored at -80°C for biochemical analysis, while the other was post-fixed in 4 % PFA for immunohistochemistry (IHC).

### Cerebrospinal fluid (CSF) collection

Mice were anesthetized and mounted onto a stereotaxic instrument. The neck skin was cut and subcutaneous tissue and muscles separated with the help of micro-retractors (Fine Science Tools, Heidelberg, Germany). Mice were then laid down so that the head formed an angle of about 135° with the body (Liu and Duff (2008) J. Vis. Exp. 10:960). A capillary tube (Borosilicate glass, B100-75-10, Sutter Instruments, Novato, California, USA) was used to punch the *dura mater* of the *cisterna magna.* CSF was collected by capillary action and transferred to 0.5 mL microtubes, immediately frozen on dry ice and stored at -80°C until use. Once thawed, samples were heated at 60°C for 5 min as described in the international application WO 2008/009868 and analyzed without further freezing-thawing cycles.

### Brain extract preparation

Brain hemispheres, frontal cortex and hippocampus of 5XFAD mice and controls were thawed, weighed and homogenized in 4 volumes of tris-saline (50 mM Tris-HCI pH = 7.4, 150 mM NaCl) with a protease inhibitor cocktail (Roche Applied Science, Meylan, France). The resulting homogenates were centrifuged at 54,000 x g for 20 minutes and supernatants (the "soluble fraction") collected and aliquoted for storage at -80°C. Pellets were resuspended by brief sonication in 10 volumes of 6 M guanidine HCI in 50 mM Tris-HCl, pH = 7.6 and centrifuged again at 26,500 x g for 20 minutes. Supernatants (the "insoluble fraction") were aliquoted and stored at -80°C (Morishima-Kawashima et al. (2000) Am. J. Pathol. 157:2093-2099).

### Quantification of Aβ₄₀ and Aβ₄₂

ELISA kits from IBL International (Hamburg, Germany) for the dosage of Aβ₄₀ (human amyloid-β (1-40) assay kit, #27713) or Aβ₄₂ (human amyloid-β (1-42) assay kit, #27719) were used according to the manufacturer's instructions. Reactions were read at 620 nm and 450 nm using an Infinite 2000 luminescence counter. The obtained values were normalized to the protein concentration of each sample, measured using a BCA protein assay (Sigma-Aldrich).

### Immunohistochemistry

Thirty-micrometer-thick sections were cut using a vibratome (Microm HM 650V, Thermo Scientific, Saint Herblain, France) and stored in cryoprotectant medium at -20°C. For the labeling of amyloid plaques, free-floating tissue sections of frontal cortex, hippocampus and entorhinal cortex (coordinates from the bregma: frontal cortex = 1.98 mm, hippocampus = -1.94 mm, entorhinal cortex = -3.08 mm) were extensively washed in PBS and then incubated in blocking solution (PBS; 3% BSA; 0.1% Triton X-100) for 1 h. Sections were stained with Hoechst dye (1:1000, Life Technologies, Saint Aubin, France) for 15 minutes to detect cell nuclei and then with freshly prepared thioflavin T solution (#T3516-5G, Sigma-Aldrich) (final concentration: 0.01 mg/ml in blocking buffer) for 15 minutes. After washing in 70 % ethanol for 5 minutes, samples were mounted on polylysine slides with coverslips. For GFAP (glial fibrillary acidic protein) staining, free-floating brain sections were blocked as before and incubated with polyclonal rabbit anti-GFAP (1:1000, Z0334, Dako, Les Ullis, France) at 4°C overnight. After thioflavin T staining and washing with 70 % ethanol, the secondary Alexafluor 594 goat anti-rabbit antibody (1:1000, A11012, Life Technologies) was added for 2 h. PBS washes and mounting were performed as described before.

### Image acquisition and analysis

Images were acquired with an Axiolmager Z1 microscope (Carl Zeiss S.A.S., Marly le Roi, France). Analysis of thioflavin T staining was performed blindly and data are presented as the mean number of particles per mm² in two to three tissue sections from the same brain area/animal (Image J software). GFAP expression was quantified using Image J software and results are expressed as area fractions.

### Novel object recognition test

The cognitive performance of mice was tested using the novel object recognition (NOR) test (Bevins and Besheer (2006) Nat. Protoc. 1:1306-1311). Animals were extensively handled during drug treatment prior to the test onset. Each day, mice were allowed to familiarize with the test room for at least 1 h prior to the test. Testing was carried out in a Plexiglas box (width: 35 cm, length: 20 cm, height: 20 cm) placed in a dimly lit room. On day 1 and 2, each mouse was habituated to the empty box for 10 min/day. On day 3, two objects (constructed out of plastic toys) were positioned in the cage, 5 cm away from the opposing walls. During the training session, each animal was placed between the two objects, facing the wall and then was allowed to explore the objects for 5 min. Mice were then returned to their home cage and 24 h later went through a 5 min test session in which one of the two (familiar) objects was replaced by a new one (novel). The whole experiment was video-recorded and object exploration [time spent by the mouse nose in contact with the object or by sniffing it at a distance ≤ 1 cm] was blindly measured. Two parameters were considered: 1) the exploration time (sec) spent by the animal interacting with the two familiar objects during the training session and 2) the exploration time spent by the animal interacting with the novel object relative to the total exploration time ([novel/(familiar + novel)] × 100) during the test. A discrimination index was also calculated ([novel - familiar]/[familiar + novel]).

### Statistical analysis

All values are expressed as the mean ± SEM. Significant effects of treatments were determined by ANOVA analysis followed by Tukey's *post hoc* tests in the case of multiple comparison groups. In all other cases, the unpaired Student's *t* test was used. For all statistical tests, P < 0.05 was considered significant. Analysis was performed with GraphPad Prism 7 (GraphPad Software, La Jolla, CA).

### Results

### Effects of Donecopride on amyloid load in 5XFAD brains

27 female 5XFAD mice (B6/SJL genetic background) were used in this study. Mice received vehicle or compounds (1 mg / kg) i.p., twice a week for two months, from 2 to 4 months of age.

Aβ₄₀ and Aβ₄₂ were quantified from brain extract in soluble and insoluble fractions. Results are expressed in % of vehicle.

The results were obtained are presented in Table 6 below:

**Table 6: Aβ₄₀ and Aβ₄₂ levels (% of controls)**

| | **Soluble fraction** | | | **Insoluble fraction** | | |
|---|---|---|---|---|---|---|
| | **A**β₄₀ | **SEM** | **n** | **A**β₄₀ | **SEM** | **n** |
| **Vehicle** | 100.00 | 11.38 | 7 | 100.00 | 14.56 | 7 |
| **Donecopride** | 110.18 | 10.68 | 7 | 79.97 | 6.40 | 7 |
| | | | | | | |

| | **A**β₄₂ | **SEM** | **n** | **A**β₄₂ | **SEM** | **n** |
|---|---|---|---|---|---|---|
| **Vehicle** | 100.00 | 11.26 | 6 | 100.00 | 6.70 | 7 |
| **Donecopride** | 65.93* | 8.36 | 7 | 67.85* | 12.59 | 6 |

| | | | | | | |
|---|---|---|---|---|---|---|
| *: p < 0.05 versus vehicle, determined by one-way ANOVA followed by a Tukey's test. | | | | | | |

The values of the vehicle groups presented in Table 6 are as follows:
∘ soluble faction:
   ▪ Aβ₄₀ = 9.48 ± 1.08 ng/mg of protein,
   ▪ Aβ₄₂ = 681.01 ± 99.13 ng/mg of protein;
∘ insoluble fraction:
   ▪ Aβ₄₀ = 0.24 ± 0.02 ng/mg of protein,
   ▪ Aβ₄₂ = 10,834.16 ± 726.36 ng/mg of protein.

**Anti-amyloid effect.** In the soluble fraction of 5XFAD mouse brains, the chronic administration of Donecopride (1 mg/kg, twice a week) was able to significantly reduce Aβ₄₂ levels (*p < 0.05 determined by one-way ANOVA followed by Tukey's test) without any change on Aβ₄₀ levels. Similarly, in the insoluble fraction, Donecopride reduced Aβ₄₂ load in the mouse brains. Insoluble Aβ₄₀ levels were unaffected by the drug.

### Effects of Donecopride on memory, amyloid load in CSF, amyloid plaques and astrogliosis in 5XFAD mice

28 female 5XFAD mice (C57BL/6J genetic background) were used in this study. Mice received vehicle or compounds (1 mg / kg) i.p., twice a week for three months, from 1 to 4 months of age.

At the end of the treatment, mice were evaluated for long-term memory performance in novel object recognition test (NOR). The inter-session interval was 24 hours. As no mice failed to explore either of the two objects or explored both objects for less than 10 sec during the training session, all the mice were included in the data analysis.

**Anti-amnesic effect.** One-month-old 5XFAD females and WT littermates were treated with RS 67333, vehicle or Donecopride (1 mg/kg, twice a week for 3 months) and then recognition memory was assessed with the novel object recognition (NOR) test. Habituation sessions were started 3 days after the end of treatment to avoid interference with acute effects. The interval between training session and test was of 24h. Vehicle-treated mice presented impaired memory performance and were not able to discriminate the novel object versus the familiar one, as shown by similar exploration time and non-significant discrimination index. RS 67333-treatment reverses cognitive impairment as indicated by the higher percentage of time devoted to the exploration of the novel object in comparison to mice treated with vehicle (**p < 0.01 versus familiar object, determined by two-way ANOVA followed by a Sidak's test). Similarly, Donecopride chronic treatments were able to prevent memory alteration (**p < 0.01 versus familiar object, determined by two-way ANOVA followed by a Sidak's test). Discrimination index of the 2 compound-treated groups were different from zero, the chance level (determined by unpaired Student's t test). The total exploration time during the training session did not differ significantly in the three groups, indicating that the recognition memory improvement in 5XFAD mice treated with Donecopride is not secondary to an increase of the exploratory activity.

**Amyloid load in CSF.** Aβ₄₂ concentration in cerebrospinal fluid (CSF) of 5XFAD mice treated with RS 67333 or Donecopride (1 mg/kg, twice a week for 3 months) was not significantly different compared to controls, indicating that chronic 5-HT₄ receptor agonist stimulation did not affect amyloid levels in CSF (determined by one-way ANOVA followed by a Tukey's test).

**Anti-amyloid effect.** Frontal cortex, hippocampus and entorhinal cortex were analyzed as representative areas of 5XFAD mouse brain (frontal, median and caudal sections) and of human brain regions that are affected early by Alzheimer's disease and are highly enriched in amyloid deposits.

Donecopride treatment (1 mg/kg, twice a week for 3 months) significantly decreased the number of amyloid plaques compared to vehicle in frontal cortex (reduction of 16 ± 3%, **p < 0.01 compared with vehicle) and in entorhinal cortex (reduction of 24 ± 4%, **p < 0.01 compared with vehicle). No significant effect was seen following RS 67333-treatment (1 mg/kg, twice a week for 3 months) neither in frontal cortex nor in entorhinal cortex. Amyloid plaque number in hippocampus of 5XFAD mice treated with RS 67333 or Donecopride (1 mg/kg, twice a week for 3 months) was not significantly different compared to controls. Statistics were determined by one-way ANOVA followed by a Tukey's test.

**Anti-inflammatory effect.** Immunohistochemical assessment of astroglial (anti-GFAP antibody, Glial fibrillary acidic protein) showed that Donecopride and RS 67333 chronic treatments (1 mg/kg, twice a week for 3 months) significantly reduced astrogliosis (54 ± 10 % and 62 ± 6 % decrease of GFAP staining in entorhinal brain slices of treated animals in comparison to controls that received vehicle, respectively, *p < 0.01 compared with vehicle). However, GFAP staining in frontal cortex or hippocampus of 5XFAD mice treated with RS 67333 or Donecopride (1 mg/kg, twice a week for 3 months) was not significantly different compared to controls. Statistics were determined by one-way ANOVA followed by a Tukey's test.

### Conclusion

The study demonstrates that a compound bearing for acetylcholinesterase inhibitory activity and 5-HT₄ receptor agonist activity (Donecopride) exerts anti-amyloid effects (reduction of Aβ₄₂ levels and amyloid plaque) and anti-inflammatory effects (reduction of astrogliosis) upon chronic administration to 5XFAD mice, a transgenic mouse model of Alzheimer's disease. These effects are mostly prominent in entorhinal cortex and frontal cortex. Moreover, chronic administration of Donecopride is able to exert an anti-amnesic effect and to prevent the appearance of memory deficits in 5XFAD mice.

All these effects are comparable, and sometimes greater, to those of RS 67333 (5-HT₄ receptor agonist chosen as reference), pointing out a combination of acetylcholinesterase inhibitory activity and 5-HT₄ receptor agonist activity as promising drug against Alzheimer's disease.

## Claims

1. Pharmaceutical composition comprising an acetylcholinesterase inhibitor and a 5-HT₄ receptor agonist, for use as neuroprotective agent in the prevention and/or treatment of a neurodegenerative disease.

2. The pharmaceutical composition for its use according to claim 1, wherein the acetylcholinesterase inhibitor is selected from the group consisting in Donepezil, rivastigmine, tacrine and galantamine.

3. The pharmaceutical composition for its use according to claim 1 or 2, wherein the acetylcholinesterase inhibitor is Donepezil.

4. The pharmaceutical composition for its use according to any one of claims 1 to 3, wherein the 5-HT₄ receptor agonist is selected from the group consisting in prucalopride, cisapride, dazopride, mosapride, renzapride, naronapride, zacopride, velusetrag, tegaserod, metoclopramide, cinitapride, YM-53389, RS-67333, 5-Methoxytryptamine (5-MT), RS-56532, ML-10302, SSP-002392, BIMU-8 and DA-6886.

5. The pharmaceutical composition for its use according to any one of claims 1 to 4, wherein the 5-HT₄ receptor agonist is RS-67333.

6. The pharmaceutical composition for its use according to any one of claims 1 to 5, wherein the neurogenerative disease is selected from the group consisting in Alzheimer's disease, Parkinson's disease, Huntington's disease, multiple system atrophy, motor neurone diseases including amyotrophic lateral sclerosis, Down syndrome and frontotemporal degenerations.

7. Neuroprotective pharmaceutical combination comprising an acetylcholinesterase inhibitor and a 5-HT₄ receptor agonist for simultaneous, separate or sequential use as neuroprotective agent in the prevention and/or treatment of a neurodegenerative disease.

8. The neuroprotective pharmaceutical combination for its use according to claim 7, wherein the acetylcholinesterase inhibitor is selected from the group consisting in Donepezil, rivastigmine, tacrine and galantamine.

9. The neuroprotective pharmaceutical combination for its use according to claim 7 or 8, wherein the acetylcholinesterase inhibitor is Donepezil.

10. The neuroprotective pharmaceutical combination for its use according to any one of claims 7 to 9, wherein the 5-HT₄ receptor agonist is selected from the group consisting in prucalopride, cisapride, dazopride, mosapride, renzapride, naronapride, zacopride, velusetrag, tegaserod, metoclopramide, cinitapride, YM-53389, RS-67333, 5-Methoxytryptamine (5-MT), RS-56532, ML-10302, SSP-002392, BIMU-8 and DA-6886.

11. The neuroprotective pharmaceutical combination for its use according to any one of claims 7 to 10, wherein the 5-HT₄ receptor agonist is RS-67333.

12. The neuroprotective pharmaceutical combination for its use according to any one of claims 7 to 11, wherein the neurogenerative disease is selected from the group consisting in Alzheimer's disease, Parkinson's disease, Huntington's disease, multiple system atrophy, motor neurone diseases including amyotrophic lateral sclerosis, Down syndrome and frontotemporal degenerations..

13. Acetylcholinesterase inhibitor for use in combination with a 5-HT₄ receptor agonist as neuroprotective agent in the prevention and/or treatment of a neurodegenerative disease.

14. 5-HT₄ receptor agonist for use in combination with an acetylcholinesterase inhibitor as neuroprotective agent in the prevention and/or treatment of a neurodegenerative disease.
